(19) Europäisches Patentamt
European Patent Office
Office européen des brevets

(11) EP 4 019 048 A1

(12) # EUROPEAN PATENT APPLICATION
published in accordance with Art. 153(4) EPC

(43) Date of publication:
**29.06.2022 Bulletin 2022/26**

(21) Application number: **20854802.4**

(22) Date of filing: **21.08.2020**

(51) International Patent Classification (IPC):
***A61K 45/00*** *(2006.01)* ***A61P 43/00*** *(2006.01)*
***A61P 17/00*** *(2006.01)* ***A61Q 15/00*** *(2006.01)*
***A61K 8/49*** *(2006.01)*

(52) Cooperative Patent Classification (CPC):
**A61K 8/49; A61K 45/00; A61P 17/00; A61P 43/00; A61Q 15/00**

(86) International application number:
**PCT/JP2020/031672**

(87) International publication number:
**WO 2021/033773 (25.02.2021 Gazette 2021/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.08.2019 US 201962890053 P**

(71) Applicant: **The University of Tokyo**
**Bunkyo-ku**
**Tokyo 113-8654 (JP)**

(72) Inventors:
- **TOYODA Yu**
  **Tokyo 113-8654 (JP)**
- **TAKADA Tappei**
  **Tokyo 113-8654 (JP)**
- **SUZUKI Hiroshi**
  **Tokyo 113-8654 (JP)**

(74) Representative: **J A Kemp LLP**
**80 Turnmill Street**
**London EC1M 5QU (GB)**

(54) **ABCC11 INHIBITOR**

(57) This invention provides an inhibitor of multidrug resistance-associated proteins with 12 transmembrane domains comprising, as an active ingredient, a compound for treatment of hyperuricemia or a salt thereof. In an embodiment, the compound is represented by Formula I. In an embodiment, the multidrug resistance-associated protein with 12 transmembrane domains is ABCC11. In particular, the inhibitor of the present invention can be used in a pharmaceutical or cosmetic composition for the prevention or treatment of axillary osmidrosis.

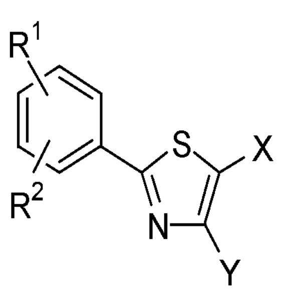

I

Fig. 4

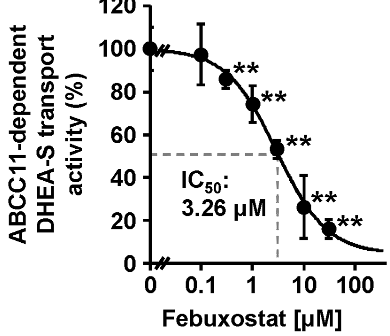


EP 4 019 048 A1

## Description

### Technical Field

The present invention relates to an inhibitor of multidrug resistance-associated proteins with 12 transmembrane domains and an ABCC11 inhibitor. The present invention also relates to a composition containing such inhibitor. More specifically, the present invention relates to an agent for the prevention and treatment of axillary osmidrosis and a pharmaceutical or cosmetic composition containing such agent.

### Background Art

Humans produce body odors like other animals. In human culture, however, strong body odors or particular body odors are often perceived unfavorable. In particular, axillary osmidrosis (hereafter, it may be referred to as "AO") is a chronic dermal disease characterized by unpleasant body odors and excessive sweating in the underarms (Non-Patent Document 1). In Asian countries, such as Japan or China, where the percentage of people with strong body odors is low, in particular, AO is more likely to be disliked and thus is recognized as a disease (Non-Patent Document 2).

The human ATP-binding cassette transporter C11 (ATP-binding cassette subfamily C member 11, ABCC11) is reported to be an AO risk factor (Non-Patent Documents 1 to 4). While the physiological substrate of ABCC11 is not completely elucidated, there is substantially no risk of AO in ABCC11-deficient subjects, and functional ABCC11 is present in the axillary apocrine glands that secrete various odor precursors. Accordingly, inhibition of ABCC11 is expected to be useful to overcome AO.

Meanwhile, use of various uric acid excretion stimulators and inhibitors of uric acid production for treatment or alleviation of acute arthritis or gout, which is associated with an underlying disease; i.e., hyperuricemia, is known. A non-purine selective xanthine oxidase inhibitor: Febuxostat, developed for the purpose of management of hyperuricemia is commercially available as a therapeutic agent for hyperuricemia from TEIJN PHARMA LIMITED (tradename: Feburic, Patent Document 1).

### Prior Art Documents

### Patent Documents

Patent Document 1: JP Patent No. 2,725,886

### Non-Patent Documents

Non-Patent Document 1: Inoue Y. et al., Journal of plastic, reconstructive & aesthetic surgery: JPRAS 2010, 63: 1369-1374

Non-Patent Document 2: Ishikawa T. et al., Frontiers in genetics 2013, 3: 306

Non-Patent Document 3: Toyoda Y. et al., FASEB journal: Official publication of the Federation of American Societies for Experimental Biology 2009, 23: 2001-2013

Non-Patent Document 4: Martin A. et al., J. Invest. Dermatol., 2010, 130: 529-540

### Summary of the Invention

### Objects to Be Attained by the Invention

Primary methods for management of AO that have been available are dermal application of alkaline substances and fragrances for neutralization and elimination of causative components of underarm odors, and dermal application of deodorants containing agents aimed at sterilization and elimination of the resident skin microbiota. While surgical treatment is performed as causal treatment of AO, such surgical treatment involves a high cost and a risk. Thus, patients with AO may hesitate to receive such active treatment.

At present, there are no agents approved for treatment of AO, and there are no ABCC11 inhibitors used in clinical situations. In addition, there is substantially no information concerning ABCC11 inhibitors.

Accordingly, development of a method for treatment of uncomfortable condition, AO, has been awaited.

Means for Attaining the Objects

The present inventors have surprisingly discovered that, as described above, a plurality of compounds including Febuxostat, which have been used for treatment of hyperuricemia, can function as ABCC11 inhibitors. In addition, the present inventors have discovered that these compounds can function as inhibitors of multidrug resistance-associated proteins with 12 transmembrane domains having structures and functions similar to those of ABCC11. Such findings led to the completion of the present invention.

Specifically, the present invention is as follows.

[1] An inhibitor of multidrug resistance-associated proteins with 12 transmembrane domains comprising, as an active ingredient, a compound for treatment of hyperuricemia or a salt thereof.

[2] An ABCC11 inhibitor comprising, as an active ingredient, a compound for treatment of hyperuricemia or a salt thereof.

[3] The inhibitor according to [1] or [2], wherein the compound is represented by Formula I:

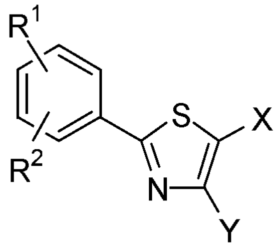

I

wherein

$R^1$ represents a halogen atom, a nitro group ($-NO_2$), a cyano group (-CN), a formyl group (-CHO), or a trifluoromethyl group ($-CF_3$),
$R^2$ represents a hydrogen atom or an alkoxy group having $C_{1-10}$ linear or branched alkyl group,
X represents a carboxyl group ($-CO_2H$), a carbamoyl group ($-CONH_2$), or an alkoxycarbonyl group having $C_{1-5}$ linear or branched alkoxy group, and
Y represents a hydrogen atom or a $C_{1-4}$ linear or branched alkyl group.

[4] The inhibitor according to [3], wherein X represents a carboxyl group and Y represents a methyl group.

[5] The inhibitor according to [3] or [4], wherein $R^2$ represents an alkoxy group having $C_{1-4}$ linear or branched alkyl group.

[6] The inhibitor according to any of [3] to [5], wherein $R^1$ represents a cyano group.

[7] The inhibitor according to [1] or [2], wherein the compound is represented by Formula II:

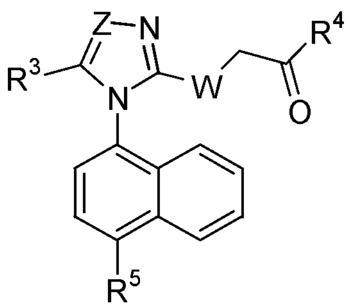

II

wherein

$R^3$ represents a hydrogen atom, a halogen atom, a cyano group (-CN), a hydroxyl group (-OH), a nitro group ($-NO_2$), or an amino group ($-NH_2$),
$R^4$ represents a hydroxyl group (-OH), an amino group ($-NH_2$), an alkoxy group having a $C_{1-6}$ linear or branched alkyl group, or a monoalkylamino or dialkylamino group substituted with a $C_{1-6}$ linear or branched alkyl group,
$R^5$ represents a cyano group (-CN), a $C_{1-6}$ linear or branched alkyl group, or a $C_{3-5}$ cycloalkyl group,

Z represents a carbon or nitrogen atom, and
W represents a sulfur or oxygen atom.

[8] The inhibitor according to [7], wherein $R^3$ represents F, Cl, or Br.
[9] The inhibitor according to [7] or [8], wherein $R^4$ represents a hydroxyl group or an alkoxy group having $C_{1-4}$ linear or branched alkyl group.
[10] The inhibitor according to any of [7] to [9], wherein $R^5$ represents a cyano or cyclopropyl group.
[11] The inhibitor according to any of [7] to [10], wherein Z represents a nitrogen atom and W represents a sulfur atom.
[12] An inhibitor of multidrug resistance-associated proteins with 12 transmembrane domains comprising, as an active ingredient, a compound selected from the group consisting of Febuxostat, Lesinurad, and a salt thereof.
[13] The inhibitor according to any of [1] to [12], which lowers a level *of in vitro* transport of small molecule substances mediated by multidrug resistance-associated proteins with 12 transmembrane domains in plasma membrane vesicles expressing multidrug resistance-associated proteins with 12 transmembrane domains to 80% or lower.
[14] A pharmaceutical or cosmetic composition comprising the inhibitor according to any of [1] to [13].
[15] The pharmaceutical or cosmetic composition according to [14], for the prevention or treatment of axillary osmidrosis.
[16] The pharmaceutical or cosmetic composition according to [15], which is a topical preparation.
[17] The pharmaceutical or cosmetic composition according to [16], in the form of an ointment, cream, emulsion, lotion, spray, powder, or gel.

This description includes part or all of the content as disclosed in the description and/or drawings of US Provisional Patent Application No. 62/890,053 filed on August 21, 2019, which is a priority document of the present application.

Effects of the Invention

According to the present invention, AO can be prevented and treated by different mechanisms from those of conventional agents and treatment methods. The inhibitor of the present invention is safe and stable, and thus is very suitably applied to humans. In addition, the present invention can provide a novel therapeutic agent against anticancer agent resistance.

Brief Description of Drawings

Fig. 1 shows ABCC11 expression on plasma membrane vesicles. $Na^+/K^+$-ATPase: loading control.
Fig. 2 shows [1,2,6,7-$^3$H(N)]-dehydroepiandrosterone sulfate (DHEA-S) transport activity in the presence or absence of ATP (n = 3). **: $P < 0.01$, NS: no significant differences (parametric Tukey-Kramer multiple comparison test, relative to other groups).
Fig. 3 shows time-dependent increase in DHEA-S transport by ABCC11 (n = 3). ++: $P < 0.01$ (two-sided t-test).
Fig. 4 shows dose-dependent inhibition of ABCC11-mediated DHEA-S transport by Febuxostat (n = 4). **: $P < 0.01$ (Dunnett' test, relative to 0 μM).
Fig. 5 shows effects of Febuxostat on ABCC2 functions. Estradiol 17β-D-glucuronide [estradiol-6,7-$^3$H(N)] ($E_2$17βG)] transport activity was measured in the presence or absence of 3.26 μM Febuxostat (FB) for 5 minutes. Data are expressed as mean ± standard deviation (n = 3). Statistical analysis was carried out using Bartlett's test and parametric Tukey-Kramer multiple comparison test. In the figure, "a," "b," and "c" each indicate the presence of a significant difference between groups ($P < 0.05$).
Fig. 6 shows effects of Alloprinol (AL), Benzbromarone (BZ), Febuxostat (FB), Lesinurad (LS), Oxypurinol (OX), and Probenecid (PR) on ABCC11-mediated DHEA-S transport (n = 4). No agents were added to the control group.
Fig. 7 shows effects of Febuxostat on ABCC2 and ABCC4 functions. ATP-dependent $E_2$17βG transport activity in ABCC2- or ABCC4-expressing plasma membrane vesicles was measured in the presence or absence of 3.26 μM Febuxostat (FB). Data are expressed as mean ± standard deviation (n = 3).
Fig. 8 shows effects of Febuxostat on ABCC2 and ABCC4 functions. ATP-dependent DHEA-S transport activity in ABCC2- or ABCC4-expressing plasma membrane vesicles was measured in the presence or absence of 3.26 μM Febuxostat (FB). Data are expressed as mean ± standard deviation (n = 3).

Embodiments of the Invention

<Inhibitor of multidrug resistance-associated proteins with 12 transmembrane domains>

The present invention provides an inhibitor of multidrug resistance-associated proteins with 12 transmembrane domains comprising, as an active ingredient, a compound for treatment of hyperuricemia or a salt thereof.

The term "multidrug resistance-associated proteins with 12 transmembrane domains" (12-transmembrane multidrug resistance-associated proteins) used herein refers to ATP-binding cassette (ABC) transporters that are localized on cell membranes and play a role in transmembrane passage of small molecule substances (Toyoda Y. et al., Xenobiotica, Jul 2008, 38 (7-8): 833-62). Specifically, the "multidrug resistance-associated proteins with 12 transmembrane domains" are selected from the group consisting of ABCC4, ABCC5, ABCC11, and ABCC12.

Including pseudogenes, ten types of ABC transporters that belong to the class of multidrug resistance-associated proteins (MRPs) have been reported. Among them, ABCC4, ABCC5, ABCC11, and ABCC12 have amino acid sequences similar to each other, compared with amino acid sequences of other multidrug resistance-associated proteins. These 4 types of transporter proteins form multidrug resistance-associated proteins with 12 transmembrane domains, and types of substances that can be transported in an ATP-dependent manner; i.e., substrate specificity, of such transporter proteins are known to be similar to each other, compared with substrate specificity of other multidrug resistance-associated proteins (Toyoda Y. et al., Xenobiotica, Jul 2008, 38 (7-8): 833-62).

The findings of the present inventors imply that the inhibitor of the present invention inhibits the transport mediated by ABCC4, ABCC5, ABCC11, and ABCC12, but does not inhibit the transport mediated by ABCC1, ABCC2, ABCC3, ABCC6, and ABCC10 belonging to a group different from the group to which ABCC11 belongs (i.e., the group of multidrug resistance-associated proteins with 17 transmembrane domains).

Accordingly, a compound for treatment of hyperuricemia, which was verified to have inhibitory activity on ABCC11 at first, or a salt thereof, can inhibit the transport of substances mediated by multidrug resistance-associated proteins with 12 transmembrane domains, and can function as an ABCC4 inhibitor, an ABCC5 inhibitor, an ABCC11 inhibitor, and/or an ABCC12 inhibitor.

In particular, ABCC4, ABCC5, and ABCC11 are observed to discharge anticancer agents to the outside of the cells. Thus, the inhibitor of the present invention can function as an auxiliary agent for overcoming multidrug resistance of cancer.

The nucleotide sequence information of ABCC4 gene is available under Gene ID: 10257 and the amino acid sequence information thereof is available under, for example, Reference Sequence: NP_005836.2 in the database of NCBI or other institutions.

The nucleotide sequence information of ABCC5 gene is available under Gene ID: 10057 and the amino acid sequence information thereof is available under, for example, Reference Sequence: NP_005679 in the database of NCBI or other institutions.

The nucleotide sequence information of ABCC11 gene is available under, for example, Gene ID: 85320 or Accession NO: NM_033151 and the amino acid sequence information thereof is available under, for example, Reference Sequence: NP_149163.2 in the database of NCBI or other institutions.

The nucleotide sequence information of ABCC12 gene is available under Gene ID: 94160 and the amino acid sequence information thereof is available under, for example, Reference Sequence: NP_150229.2 in the database of NCBI or other institutions.

In the present description, the expression "inhibition of multidrug resistance-associated proteins with 12 transmembrane domains" refers to lowering or quenching of functions of multidrug resistance-associated proteins with 12 transmembrane domains *in vitro* or *in vivo,* and it may refer to, for example, lowering or quenching of the transport or secretion of small molecule substances mediated by multidrug resistance-associated proteins with 12 transmembrane domains. The level of lowering is not particularly limited, provided that it is statistically significant. For example, the amount of transport or secretion can be lowered to 80% or lower, 70% or lower, 60% or lower, 50% or lower, or 40% or lower than that in the absence of a compound that is predicted to have inhibitory effects on multidrug resistance-associated proteins with 12 transmembrane domains.

The "small molecule substances" mediated to secrete by multidrug resistance-associated proteins with 12 transmembrane domains are not particularly limited. Examples thereof include androgen steroids, such as dehydroepiandrosterone sulfate (DHEA-S) and estrone-3-sulfate, cyclic nucleotides, such as cAMP and cGMP, glutathione conjugates, such as leucotriene C4 and S-(2,4-dinitrophenyl)-glutathione, glucuronic acid conjugates, such as estradiol 17-β-D-glucuronide and bile salt, anticancer agents, such as 5-fluorouracil (5-FU) and methotrexate (MTX), and antivirus agents, such as 9-(2-phosphonyl-methoxyethyl)adenine (PMEA).

The term "compound for treatment of hyperuricemia" used herein refers to a substance that exerts or is expected to exert activity of lowering serum uric acid level. Such substance has inhibitory activity on uric acid generating enzymes or inhibitory activity on uric acid transporter.

<ABCC11 inhibitor>

The present invention also provides an ABCC11 inhibitor comprising, as an active ingredient, a compound for treatment of hyperuricemia or a salt thereof.

The present inventors' group has reported that the risk of axillary osmidrosis (AO) is determined by non-synonymous single nucleotide polymorphism in ABCC11 gene (c.538G>A (p.Gly180Arg)) (Inoue Y. et al., Journal of plastic, reconstructive & aesthetic surgery: JPRAS 2010, 63: 1369-1374; Toyoda Y. et al., FASEB journal: Official publication of the Federation of American Societies for Experimental Biology 2009, 23: 2001-2013; Martin A. et al., J. Invest. Dermatol., 2010, 130: 529-540). Biochemical analysis demonstrates that this amino acid substitution (variant type: Arg180) would promote proteasome decomposition of *de novo* synthesized ABCC11 protein, resulting in the loss of intracellular functions of ABCC11. Functional ABCC11 (wild type: Gly180) is present in axillary apocrine glands that secrete a wide variety of odor precursors (Toyoda Y. et al., Int. J. Mol. Sci., 2017, 18: 417). Accordingly, inhibition of ABCC11 functions can contribute to prevention and treatment of AO.

As described above, ABCC11 is one of ATP-binding cassette (ABC) transporters which are localized on cell membranes and play a role in transmembrane passage of small molecule substances (Toyoda Y. et al., Xenobiotica, Jul 2008, 38 (7-8): 833-62).

The term "inhibition of ABCC11" used herein refers to lowering or quenching of functions of the wild-type (functional) ABCC11 *in vitro* or *in vivo,* and it may refer to, for example, lowering or quenching of the transport or secretion of small molecule substances mediated by ABCC11. The level of lowering is not particularly limited, provided that it is statistically significant. For example, the amount of transport or secretion can be lowered to 80% or lower, 70% or lower, 60% or lower, 50% or lower, or 40% or lower than that in the absence of a compound that is predicted to have inhibitory effects on ABCC11. The "small molecule substances" mediated to secrete by ABCC11 are not particularly limited. Examples thereof include androgen steroids, such as dehydroepiandrosterone sulfate (DHEA-S) and estrone-3-sulfate, cyclic nucleotides, such as cAMP and cGMP, glutathione conjugates, such as leucotriene C4 and S-(2,4-dinitrophenyl)-glutathione, glucuronic acid conjugates, such as estradiol 17-β-D-glucuronide and bile salt, anticancer agents, such as 5-fluorouracil (5-FU) and methotrexate (MTX), and antivirus agents, such as 9-(2-phosphonyl-methoxyethyl)adenine (PMEA).

Accordingly, the ABCC11 inhibitor of the present invention can be suitably used for the prevention or treatment of axillary osmidrosis by lowering or quenching the secretion of hircismus-causing substances mediated by functional ABCC11. However, the ABCC11 inhibitor of the present invention can exert other advantageous effects by lowering or quenching other functions mediated by ABCC11, such as the transport of other small molecule substances. For example, it has been reported that cells highly expressing ABCC11 would develop resistance to anticancer agents such as 5-FU in the case of lung cancer (Uemura, T. et al., Cancer Sci., 101 (11): 2404-2410, 2010). It is thus considered that the ABCC11 inhibitor may be used for potentiating effects of anticancer agents.

Unlike humans, rodents such as mice and rats do not have Abcc11 gene. Accordingly, it is substantially impractical to investigate physiological influence of ABCC11 inhibition by *in vivo* experiments. The present inventors have developed a technique of *in vitro* transport assay suitable for searching ABCC11 inhibitor with the use of ABCC11-expressing plasma membrane vesicles (Toyoda Y. et al., Journal of Dermatology 2020, doi: 10.1111/1346-8138.15512).

Briefly, plasma membrane vesicles were prepared from adenovirus-mediated transiently ABCC11-expressing 293A cells, and an ABCC11 substrate [1,2,6,7-$^{3}$H(N)]-dehydroepiandrosterone sulfate (DHEA-S) was subjected to immunoblotting and *in vitro* transport assay. As shown in Fig. 1, the prepared vesicles express ABCC11, and can perform ABCC11-mediated ATP-dependent DHEA-S transport into vesicles (Fig. 2). The DHEA-S transport was found to increase over time (Fig. 3).

Subsequently, the ABCC11-mediated DHEA-S transport activity was inspected in the presence of various compounds. Surprisingly, the results demonstrate that the transport is inhibited in a dose-dependent manner in the presence of compounds known to be used for treatment of hyperuricemia and that, in particular, Febuxostat and Lesinurad exhibit high effects, identifying these compounds as novel ABCC11 inhibitors. In consideration of interactions with ABCC11 protein, in particular, analog compounds of Febuxostat and Lesinurad can have similar activity.

The present inventors also discovered that Alloprinol, Benzbromarone, Oxypurinol, and Probenecid, that are known to exert therapeutic effects on hyperuricemia, can inhibit ABCC11-mediated DHEA-S transport activity.

In addition, the present inventors discovered that the compounds inhibiting ABCC11-mediated transport of substances do not inhibit the transport of substances mediated by ABCC2, belonging to the group of multidrug resistance-associated proteins with 17 transmembrane domains, but inhibit the transport of small molecule substances mediated by ABCC4, belonging to the group of multidrug resistance-associated proteins with 12 transmembrane domains to which ABCC11 also belongs. In consideration of similarity in structures and functions of the group of multidrug resistance-associated proteins with 12 transmembrane domains and differences in substrates resulting from the differences from the group of multidrug resistance-associated proteins with 17 transmembrane domains, the inhibition mechanisms of the inhibitor of the present invention are considered to be common in the group of multidrug resistance-associated

proteins with 12 transmembrane domains.

An embodiment of the present invention provides an inhibitor of multidrug resistance-associated proteins with 12 transmembrane domains or an ABCC11 inhibitor comprising, as an active ingredient, a compound represented by Formula I or a salt thereof:

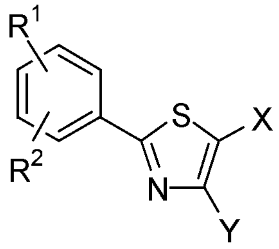

I

wherein

$R^1$ represents a halogen atom, a nitro group ($-NO_2$), a cyano group (-CN), a formyl group (-CHO), or a trifluoromethyl group ($-CF_3$),
$R^2$ represents a hydrogen atom or an alkoxy group having $C_{1-10}$ linear or branched alkyl group,
X represents a carboxyl group ($-CO_2H$), a carbamoyl group ($-CONH_2$), or an alkoxycarbonyl group having $C_{1-5}$ linear or branched alkyl group, and
Y represents a hydrogen atom or a $C_{1-4}$ linear or branched alkyl group.

A compound represented by Formula I in which X represents a carboxyl group and Y represents a methyl group is preferable, although the compound is not particularly limited thereto.

For example, a compound represented by Formula I in which $R^2$ represents an alkoxy group having a $C_{1-4}$ linear or branched alkyl group is preferable.

Alternatively, a compound represented by Formula I in which $R^1$ represents a cyano group is preferable.

An example of particularly preferable compounds represented by Formula I is Febuxostat represented by the formula below or a salt thereof.

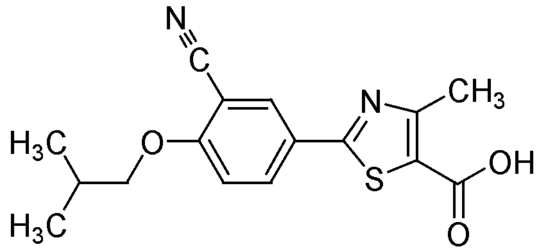

Another embodiment of the present invention provides an inhibitor of multidrug resistance-associated proteins with 12 transmembrane domains or an ABCC11 inhibitor comprising, as an active ingredient, a compound represented by Formula II or a salt thereof:

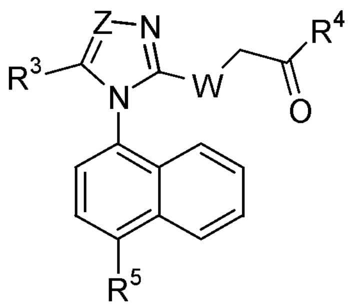

II

wherein

$R^3$ represents a hydrogen atom, a halogen atom, a cyano group (-CN), a hydroxyl group (-OH), a nitro group ($-NO_2$), or an amino group ($-NH_2$),
$R^4$ represents a hydroxyl group (-OH), an amino group ($-NH_2$), an alkoxy group having a $C_{1-6}$ linear or branched alkyl group, or a monoalkylamino or dialkylamino group substituted with a $C_{1-6}$ linear or branched alkyl group,
$R^5$ represents a cyano group (-CN), a $C_{1-6}$ linear or branched alkyl group, or a $C_{3-5}$ cycloalkyl group,
Z represents a carbon or nitrogen atom, and
W represents a sulfur or oxygen atom.

A compound represented by Formula II in which $R^3$ represents a halogen atom or, in particular, F, Cl, or Br is preferable, although the compound is not particularly limited thereto.
A compound represented by Formula II in which $R^4$ represents a hydroxyl group or an alkoxy group having a $C_{1-4}$ linear or branched alkyl group is preferable, although the compound is not particularly limited thereto.
A compound represented by Formula II in which $R^5$ represents a cyano or cyclopropyl group is also preferable, although the compound is not particularly limited thereto.
A compound represented by Formula II in which Z represents a nitrogen atom and W represents a sulfur atom is also preferable, although the compound is not particularly limited thereto.
An example of particularly preferable compounds represented by Formula II is Lesinurad represented by the formula below or a salt thereof.

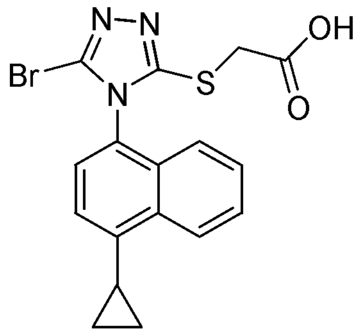

Accordingly, the present invention also provides an inhibitor of multidrug resistance-associated proteins with 12 transmembrane domains or an ABCC11 inhibitor comprising, as an active ingredient, a compound selected from the group consisting of Febuxostat, Lesinurad, and a salt thereof.
Salts of compounds for treatment of hyperuricemia including compounds represented by Formula I and Formula II are not particularly limited, provided that such salts are pharmaceutically acceptable. Examples thereof include sodium salt, potassium salt, magnesium salt, calcium salt, aluminum salt, zinc salt, ammonium salt, hydrochloride, sulfate, acetate, benzoate, and trifluoroacetic acetate.
The inhibitor of the present invention can lower the amount of apocrine gland secretory substances in individuals expressing wild-type (functional) ABCC11 protein to, for example, 80% or lower, 70% or lower, 60% or lower, 50% or lower, or 40% or lower than that in the absence of the inhibitor, although the level of inhibition is not limited thereto.
Such effects can be easily confirmed by the *in vitro* transport assays developed by the present inventors. Specifically, the inhibitor of the present invention can lower a level of ABCC11-mediated *in vitro* transport of small molecule substances through plasma membrane vesicles expressing wild-type ABCC11 protein to 80% or lower, 70% or lower, 60% or lower, 50% or lower, or 40% or lower.
The inhibitor of the present invention can also lower a level of *in vitro* transport of small molecule substances mediated by the multidrug resistance-associated proteins with 12 transmembrane domains through plasma membrane vesicles expressing the multidrug resistance-associated proteins with 12 transmembrane domains to 80% or lower, 70% or lower, 60% or lower, 50% or lower, or 40% or lower.
Small molecule substances that can be used to verify the effects by *in vitro* transport assays may be naturally-occurring or synthetic compounds that can be transported via ABCC4, ABCC5, ABCC11, and/or ABCC12 and such substances are not particularly limited. For example, [1,2,6,7-$^3$H(N)]-dehydroepiandrosterone sulfate (DHEA-S) or estradiol 17β-D-glucuronide [estradiol-6,7-$^3$H(N)] ($E_2$17βG) used in the examples below can be suitably used.
The inhibitor of the present invention can be used as, for example, an auxiliary agent for overcoming multidrug resistance of cancer. In particular, the inhibitor of the present invention can be preferably used for the prevention or treatment of axillary osmidrosis.

<Pharmaceutical composition/cosmetic composition>

The present invention also provides a pharmaceutical or cosmetic composition comprising the ABCC11 inhibitor or the inhibitor of multidrug resistance-associated proteins with 12 transmembrane domains of the present invention. Pharmaceutical compositions encompass pharmaceutical products and quasi-drugs comprising, as an active ingredient, the ABCC11 inhibitor. The pharmaceutical composition of the present invention can be a composition used for the prevention or treatment of, for example, axillary osmidrosis. The cosmetic composition of the present invention can be an antiperspirant or deodorant product comprising the ABCC11 inhibitor. The pharmaceutical composition of the present invention can be a pharmaceutical composition used for treatment or prevention of cancer comprising, as an active ingredient, the inhibitor of multidrug resistance-associated proteins with 12 transmembrane domains.
The composition of the present invention can be administered via any suitable route. For example, the composition of the present invention can be administered orally in the form of a tablet, a powder, or a liquid. The composition of the present invention can be administered parenterally by means of systemic or topical administration, such as intravenous, intramuscular, intraperitoneal, or intratumoral injection or infusion. Human axillary apocrine glands open into the hair follicles. When prevention or treatment of axillary osmidrosis is intended or antiperspirant or deodorant effects are to be attained, accordingly, topical administration to the skin, such as armpits, is effective. It is accordingly preferable that the composition be for external use.
When the pharmaceutical composition and the cosmetic composition are used as compositions for external use, any forms generally used in the art may be used. Examples of forms include, but are not particularly limited to, an ointment, a cream, an emulsion, a lotion, a spray, a powder, and a gel. When the composition is applied or sprayed, it can be applied to sites where body odors may be produced by sweating or other reasons, such as the neck, the back, hands and feet, the soles, the head, or the pubic region, as well as the armpits.
The composition of the present invention can be supplemented with, in addition to the ABCC11 inhibitor, other effective ingredients for the prevention or treatment of axillary osmidrosis, such as bactericidal agents, antibacterial agents, alkaline substances, or fragrances. Also, the composition of the present invention can be adequately supplemented with, for example, an excipient, a buffer, a surfactant, an antioxidant, a preservative, an ultraviolet absorber, or a powder, in accordance with the form of the composition, although additives are not limited thereto. When used for treatment or prevention of cancer, the composition of the present invention can comprise the inhibitor of multidrug resistance-associated proteins with 12 transmembrane domains alone or in combination with, for example, an anticancer agent.
The inhibitor, which is an active ingredient of the pharmaceutical composition of the present invention, is a safe and stable agent that is approved for use in humans as represented by Febuxostat and Lesinurad, which have been approved for clinical use.
Upon oral administration, the maximum unbound concentration of Febuxostat in plasma is 0.126 μM when 40 mg of Febuxostat is administered to a healthy subject once a day for consecutive 12 days (Khosravan R. et al., Clin. Pharmacokinet. 2006, 45: 821-841). However, a higher dose may be required in order for the orally administered Febuxostat to exert effects of prevention or treatment on axillary osmidrosis in the armpits.
The composition of the present invention can comprise the ABCC11 inhibitor of the present invention in the range of 0.001 to 10% by weight, preferably 0.01 to 3% by weight, and more preferably 0.1 to 1% by weight, based on the total weight of the composition, although the amount is not limited thereto.
When the composition of the present invention is used in the form of a composition for external use, for example, 10 mg to 10 g, and preferably 50 mg to 5 g of the composition may be applied by general means, such as dermal application or spraying, to armpits once or several instances a day. Thus, preferable effects can be expected. It should be noted that such amount varies depending on dosage forms and is not particularly limited.

<Method>

The present invention also provides a method for treatment or prevention of axillary osmidrosis, comprising administering the ABCC11 inhibitor of the present invention or the composition of the present invention comprising such inhibitor to a subject. In addition, the present invention provides an antiperspirant or deodorizing method, comprising administering the ABCC11 inhibitor of the present invention or the composition of the present invention to a subject. In the present invention, the subject is not particularly limited. The subject can be a human, and, in particular, the subject can be a human expressing wild-type (functional) ABCC11.
The present invention also provides a method for the prevention or treatment of a disease, comprising administering the inhibitor of multidrug resistance-associated proteins with 12 transmembrane domains of the present invention or the composition of the present invention comprising such inhibitor to a subject. While the disease is not particularly limited, the disease can be, for example, cancers with anticancer drug resistance in the subject.

Examples

Hereafter, the present invention is described in greater detail with reference to the examples, although the present invention is not limited to these examples. In the following examples, commercially available chemical substances of the analytical grades were used, unless otherwise specified.

All the statistical analyses were performed using Excel 2019 in combination with Statcel4 add-in software (OMS Publishing Inc., Saitama, Japan). When a plurality of groups are to be analyzed, the similarity of variance across groups was compared by the Bartlett's test. When the test for homogeneity of variance is passed, all the paired comparisons were performed by the parametric Tukey-Kramer multiple comparison test or by the Dunnett' test for comparison with the control group. When a pair of quantification data is analyzed, the variance of data sets was compared by the F test, and an unpaired student's t-test was performed. Statistical significance was defined in terms of P values less than 0.05 or 0.01.

[Example 1: Preparation of ABCC11-expressing plasma membrane vesicles]

Human embryonic kidney cells 293 (HEK293)-derived 293A cells (R70507, Invitrogen) were maintained in Dulbecco's modified Eagle medium (Nacalai Tesque Inc., Kyoto, Japan) supplemented with 10% fetal bovine serum (Biowest, Nuaille, France), 1% penicillin-streptomycin (Nacalai Tesque Inc.), 2 mM L-glutamine (Nacalai Tesque Inc.), and 1x non-essential amino acid (Life Technologies, Tokyo, Japan) at 37°C in a humidified atmosphere of 5% (v/v) $CO_2$ in air.

The 293A cells were infected with the ABCC11-expressing or EGFP-expressing (control) adenoviruses prepared in the manner described in Toyoda Y. et al., International Journal of Molecular Sciences 2017, 18: 417, and plasma membrane vesicles were then prepared in the manner described in Toyoda Y. et al., International Journal of Molecular Sciences 2017, 18: 417.

The plasma membrane vesicles obtained were rapidly frozen in liquid nitrogen and stored at -80°C before use. The protein concentration of the plasma membrane vesicles was quantified using the BCA protein assay kit (Pierce, Rockford, IL, U.S.A.) with bovine serum albumin as a standard in accordance with the manufacturer's instructions.

ABCC11 protein expression in plasma membrane vesicles was inspected by immunoblotting as described in Toyoda Y. et al., International Journal of Molecular Sciences 2017, 18: 417 and Toyoda Y. et al., FASEB journal: Official publication of the Federation of American Societies for Experimental Biology 2009, 23: 2001-2013 with minor modifications.

Briefly, the prepared sample was electrophoretically separated on polyacrylamide gel and then transferred onto the Hybond® ECL™ nitrocellulose membrane (GE Healthcare, Buckinghamshire, UK) by electroblotting at 15 V for 70 minutes.

For blocking, the membrane was subjected to incubation in Tris-buffered saline containing 0.05% Tween 20 and 5% skim milk (TBST skim milk) at 4°C overnight.

Blots were probed with the rat monoclonal anti-ABCC11 antibody (M8I-74; Abcam, Cambridge, MA, USA; 200-fold diluted) and with the rabbit polyclonal anti-$Na^+/K^+$-ATPase $\alpha$ antibody (sc-28800, Santa Cruz Biotechnology, Santa Cruz, CA, U.S.A., 1000-fold diluted), followed by incubation with the goat anti-rat immunoglobulin G (IgG)-horseradish peroxidase (HRP)-conjugated antibody (NA935V, GE Healthcare, 2000-fold diluted) and the donkey antirabbit IgG-HRP-conjugated antibody (NA934V, GE Healthcare, 3000-fold diluted), respectively. All antibodies were used in TBST skim milk.

HRP-dependent luminescence was developed with the use of the ECL™ Prime Western Blotting Detection Reagent (GE Healthcare) and detected with the use of a multiimaging analyzer Fusion Solo 4™ system (Vilber Lourmat, Eberhardzell, Germany).

As a result, ABCC11 expression was observed in plasma membrane vesicles derived from the 293A cells infected with ABCC11-expressing adenoviruses, as shown in Fig. 1. In contrast, ABCC11 expression was not observed in plasma membrane vesicles (Mock) derived from the cells infected with EGFP-expressing (control) adenoviruses. The $Na^+/K^+$-ATPase expression levels were found to be equivalent in both vesicles.

[Example 2: Vesicle transport assay]

The *in vitro* experiment to study the transport of an ABCC11 substrate: [1,2,6,7-$^3$H(N)]-dehydroepiandrosterone sulfate (DHEA-S) (PerkinElmer, Waltham, MA, U.S.A.), into the ABCC11-expressing plasma membrane vesicles and into the control plasma membrane vesicles was performed in accordance with the rapid filtration technique described in Toyoda Y. et al., International Journal of Molecular Sciences 2017, 18: 417 with minor modifications.

Briefly, plasma membrane vesicles were subjected to incubation together with 100 nM [1,2,6,7-$^3$H(N)]-DHEA-S in a reaction mixture (10 mM Tris/HCl, 250 mM sucrose, 10 mM $MgCl_2$, 10 mM creatine phosphate, 1 mg/ml creatine

phosphokinase, and 50 mM ATP or AMP as an alternative to ATP, pH 7.4) at 37°C for 5 minutes, and radioactivity derived from the incorporated DHEA-S was measured.

In order to decrease background signals derived from radiolabeled DHEA-S that had nonspecifically adsorbed to a filter membrane for plasma membrane vesicle trapping (MF-Millipore Membrane, HAWP02500; pore diameters: 0.45 μm and 25 mm; Millipore, Tokyo Japan), the filter membrane was subjected to incubation before use with 2 μM cholesterol (Wako Pure Chemical Industries, Ltd., Tokyo, Japan) in ice-cooled Stop buffer (250 mM sucrose, 0.1 M NaCl, 2 mM EDTA, and 10 mM Tris-HCl, pH 7.4) containing 0.2% (v/v) DMSO.

The results demonstrate that ATP-dependent DHEA-S transport takes place in ABCC11-expressing plasma membrane vesicles, as shown in Fig. 2.

[Example 3: Observation of DHEA-S transport over time]

In the same manner as in Example 2, ATP-dependent DHEA-S transport into the ABCC11-expressing plasma membrane vesicles and into the control plasma membrane vesicles was observed over time (incubation for 2.5 minutes, 5 minutes, 10 minutes, and 20 minutes). The level of ATP-dependent DHEA-S transport was determined by subtracting the DHEA-S transport activity in the absence of ATP from the DHEA-S transport activity in the presence of ATP.

The results demonstrate that the level of DHEA-S transport is increased over time in the ABCC11-expressing plasma membrane vesicles, as shown in Fig. 3.

[Example 4: Effects of Febuxostat for inhibiting ABCC11]

The present inventors have verified the effects of Febuxostat for inhibiting ABCC11 at the preliminary screening stage (the data are not shown). Thus, they inspected the effects ofFebuxostat (0.1 to 30 μM) for dose-dependent inhibition on ABCC11. ABCC11-dependent DHEA-S transport activity was determined by subtracting the ATP-dependent DHEA-S transport activity in the control plasma membrane vesicles from the transport activity in the ABCC11-expressing plasma membrane vesicles. The DHEA-S transport activity was measured in the presence of Febuxostat at the designated concentration for 5 minutes.

In this example, transport activity of each group was determined as the incorporated clearance [ml/mg protein/min = incorporated level of DHEA-S (disintegration per minute (DPM)/mg protein/min)/DHEA-S level in incubation mixture (DPM/ml)] and the activity was expressed as a percentage relative to the transport activity (100%) in the absence of Febuxostat (0 μM).

Based on the calculated values, fitting curves were obtained according to the following formula using the least-squares methods with the Excel 2019 (Microsoft, Redmond, WA, U.S.A.) (Chen ZS. et al., Mol. Pharmacol., 2005; 67: 545-557).

$$\text{Predicted value [\%]} = 100 - ((E_{max} \times C^n) / (EC_{50}{}^n + C^n))$$

In the formula, $E_{max}$ is the maximum effect, $EC_{50}$ is the half maximal effective concentration, C is the concentration of the test compound, and n is the sigmoid-fit factor.

In the end, the $IC_{50}$ was calculated based on the results.

As a result, Febuxostat was found to inhibit ABCC 11-dependent DHEA-S transport activity in a dose-dependent manner and the $IC_{50}$ was determined to be 3.26 μM, as shown in Fig. 4.

[Example 5: Effects on ABCC2-dependent transport]

In order to determine as to whether or not the inhibitory effects of Febuxostat is specific to ABCC11, whether or not Febuxostat would be effective on functions of ABCC2 (also known as "MRP2"), which is an ATP-binding cassette transporter different from ABCC11 was examined.

To this end, incubation was carried out at 37°C for 5 minutes with the use of human ABCC2-expressing plasma membrane vesicles, the functions of which had been verified in previous research (Toyoda Y. et al., Scientific Reports 2016, 6: 24586), control plasma membrane vesicles (Genomembrane Co., Ltd., Yokohama, Japan), and, as the ABCC2 substrate, estradiol 17β-D-glucuronide [estradiol-6,7-$^3$H(N)] ($E_2$17βG) (PerkinElmer) (100 nM in the reaction mixture), in the presence or absence of Febuxostat (FB) at 3.26 μM determined as the $IC_{50}$ value in Example 4. Other conditions were in accordance with Example 2.

The results demonstrate that ATP-dependent $E_2$17βG transport was observed in the ABCC2-expressing plasma membrane vesicles, as shown in Fig. 5, although ABCC2 inhibition by Febuxostat was not observed.

[Example 6: ABCC11 inhibitory activity of various compounds]

In the same manner as in Example 4, whether or not Alloprinol (AL), Benzbromarone (BZ), Febuxostat (FB), Lesinurad (LS), Oxypurinol (OX), and Probenecid (PR) would exert inhibitory effects on ABCC11-dependent DHEA-S transport activity was examined.

Briefly, plasma membrane vesicles were subjected to incubation together with 100 nM [1,2,6,7-$^3$H(N)]-DHEA-S in a reaction mixture (10 mM Tris/HCl, 250 mM sucrose, 10 mM $MgCl_2$, 10 mM creatine phosphate, 1 mg/ml creatine phosphokinase, and 50 mM ATP or AMP as an alternative to ATP, pH 7.4) containing compounds at the final concentration of 3 μM each at 37°C for 5 minutes, and radioactivity derived from the incorporated DHEA-S was measured. The transport activity of each group was determined as the incorporated clearance [ml/mg protein/min = incorporated level of DHEA-S (disintegration per minute (DPM)/mg protein/min)/DHEA-S level in incubation mixture (DPM/ml)], and expressed as a percentage relative to the transport activity (100%) in the absence of test compound (0 μM).

The results demonstrate that Alloprinol, Benzbromarone, Lesinurad, Oxypurinol, and Probenecid that are known to have therapeutic effects on hyperuricemia inhibit ABCC11-mediated DHEA-S transport activity as with Febuxostat, as shown in Fig. 6, and Alloprinol, Benzbromarone, Lesinurad, Oxypurinol, and Probenecid lowered the transport activity to approximately 80%, compared to the case without the addition of compounds (control). In particular, Lesinurad was found to have inhibitory activity equivalent to that of Febuxostat and, to lower the transport activity to approximately 30% of the activity of the control at 3 μM.

[Example 7: Effects on ABCC4-dependent transport]

It was verified in Example 5 that ATP-dependent $E_2$17βG transport in ABCC2-expressing plasma membrane vesicles is not inhibited by Febuxostat.

Thus, whether or not Febuxostat would be effective on functions of ABCC4 (also known as MRP4), which has been reported to have the structure and functions similar to those of ABCC11, was examined. ABCC4 can transport both the ABCC11 substrate DHEA-S and the ABCC2 substrate $E_2$17βG in an ATP-dependent manner.

Incubation was carried out using human ABCC2-expressing plasma membrane vesicles, human ABCC4-expressing plasma membrane vesicles, and control plasma membrane vesicles (obtained from Genomembrane Co., Ltd.) and, as substrates, estradiol 17β-D-glucuronide [estradiol-6,7-$^3$H(N)] ($E_2$17βG) (PerkinElmer) and [1,2,6,7-$^3$H(N)]-dehydroepiandrosterone sulfate (DHEA-S) (PerkinElmer) (100 nM in the reaction mixture) in the presence (FB) or absence (Non) of 3.26 μM Febuxostat at 37°C for 5 minutes. Other conditions are in accordance with Example 2.

The results demonstrate that, as shown in Fig. 7 and in Fig. 8, ATP-dependent $E_2$17βG transport and DHEA-S transport were observed in ABCC4-expressing plasma membrane vesicles. In the presence of Febuxostat, however, both transport activities were inhibited. In ABCC2-expressing plasma membrane vesicles, in contrast, ABCC2-mediated $E_2$17βG transport was not inhibited by Febuxostat, as demonstrated in Example 5 (Fig. 7). In ABCC2-expressing plasma membrane vesicles, ATP-dependent DHEA-S transport activity was not observed (Fig 8).

All publications, patents, and patent applications cited herein are incorporated herein by reference in their entirety.

## Claims

1. An inhibitor of multidrug resistance-associated proteins with 12 transmembrane domains comprising, as an active ingredient, a compound for treatment of hyperuricemia or a salt thereof.

2. An ABCC11 inhibitor comprising, as an active ingredient, a compound for treatment of hyperuricemia or a salt thereof.

3. The inhibitor according to Claim 1 or 2, wherein the compound is represented by Formula I:

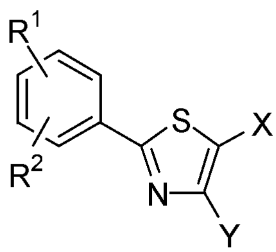

I

wherein

$R^1$ represents a halogen atom, a nitro group ($-NO_2$), a cyano group (-CN), a formyl group (-CHO), or a trifluoromethyl group ($-CF_3$),
$R^2$ represents a hydrogen atom or an alkoxy group having $C_{1-10}$ linear or branched alkyl group,
X represents a carboxyl group ($-CO_2H$), a carbamoyl group ($-CONH_2$), or an alkoxycarbonyl group having $C_{1-5}$ linear or branched alkoxy group, and
Y represents a hydrogen atom or a $C_{1-4}$ linear or branched alkyl group.

**4.** The inhibitor according to Claim 3, wherein X represents a carboxyl group and Y represents a methyl group.

**5.** The inhibitor according to Claim 3 or 4, wherein $R^2$ represents an alkoxy group having $C_{1-4}$ linear or branched alkyl group.

**6.** The inhibitor according to any one of Claims 3 to 5, wherein $R^1$ represents a cyano group.

**7.** The inhibitor according to Claim 1 or 2, wherein the compound is represented by Formula II:

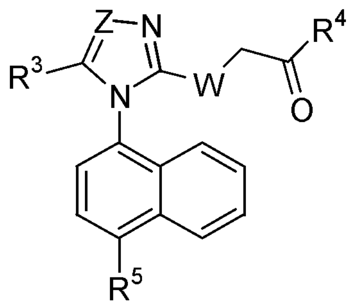

II

wherein

$R^3$ represents a hydrogen atom, a halogen atom, a cyano group (-CN), a hydroxyl group (-OH), a nitro group ($-NO_2$), or an amino group ($-NH_2$),
$R^4$ represents a hydroxyl group (-OH), an amino group ($-NH_2$), an alkoxy group having a $C_{1-6}$ linear or branched alkyl group, or a monoalkylamino or dialkylamino group substituted with a $C_{1-6}$ linear or branched alkyl group,
$R^5$ represents a cyano group (-CN), a $C_{1-6}$ linear or branched alkyl group, or a $C_{3-5}$ cycloalkyl group,
Z represents a carbon or nitrogen atom, and
W represents a sulfur or oxygen atom.

**8.** The inhibitor according to Claim 7, wherein $R^3$ represents F, Cl, or Br.

**9.** The inhibitor according to Claim 7 or 8, wherein $R^4$ represents a hydroxyl group or an alkoxy group having $C_{1-4}$ linear or branched alkyl group.

**10.** The inhibitor according to any one of Claims 7 to 9, wherein $R^5$ represents a cyano or cyclopropyl group.

**11.** The ABCC11 inhibitor according to any one of Claims 7 to 10, wherein Z represents a nitrogen atom and W represents a sulfur atom.

**12.** An inhibitor of multidrug resistance-associated proteins with 12 transmembrane domains comprising, as an active ingredient, a compound selected from the group consisting of Febuxostat, Lesinurad, and a salt thereof.

**13.** The inhibitor according to any one of Claims 1 to 11, which lowers a level *of in vitro* transport of small molecule substances mediated by multidrug resistance-associated proteins with 12 transmembrane domains in plasma membrane vesicles expressing multidrug resistance-associated proteins with 12 transmembrane domains to 80% or lower.

**14.** A pharmaceutical or cosmetic composition comprising the inhibitor according to any one of Claims 1 to 13.

15. The pharmaceutical or cosmetic composition according to Claim 14, for the prevention or treatment of axillary osmidrosis.

16. The pharmaceutical or cosmetic composition according to Claim 15, which is a topical preparation.

17. The pharmaceutical or cosmetic composition according to Claim 16, in the form of an ointment, cream, emulsion, lotion, spray, powder, or gel.

Fig. 1

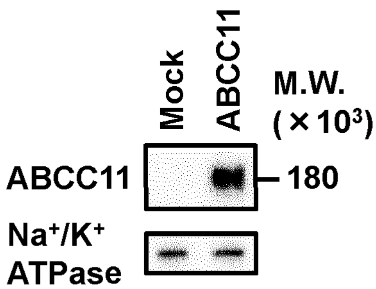

Fig. 2

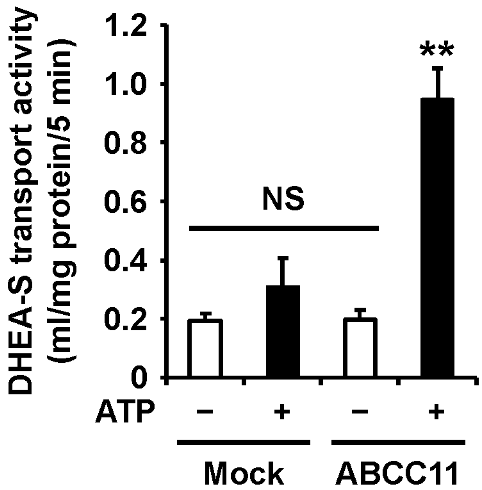
DHEA-S transport activity
(ml/mg protein/5 min)
1.2
1.0
0.8
0.6
0.4
0.2
0
**
NS
ATP
−
+
−
+
Mock
ABCC11

Fig. 3

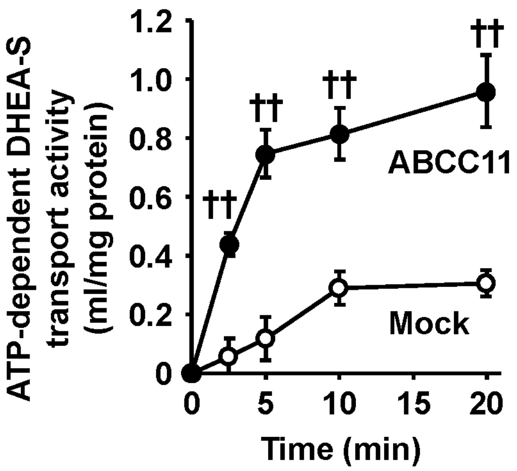
ATP-dependent DHEA-S transport activity (ml/mg protein)
1.2
1.0
0.8
0.6
0.4
0.2
0
0
5
10
15
20
Time (min)
††
††
††
††
ABCC11
Mock

Fig. 4

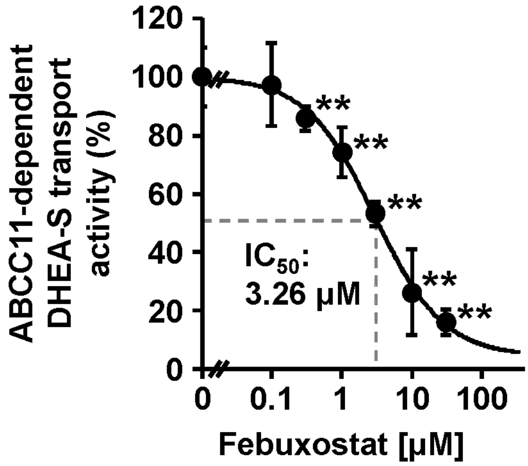
ABCC11-dependent DHEA-S transport activity (%)
120
100
80
60
40
20
0
**
**
**
**
**
IC50:
3.26 µM
0
0.1
1
10
100
Febuxostat [µM]

Fig. 5

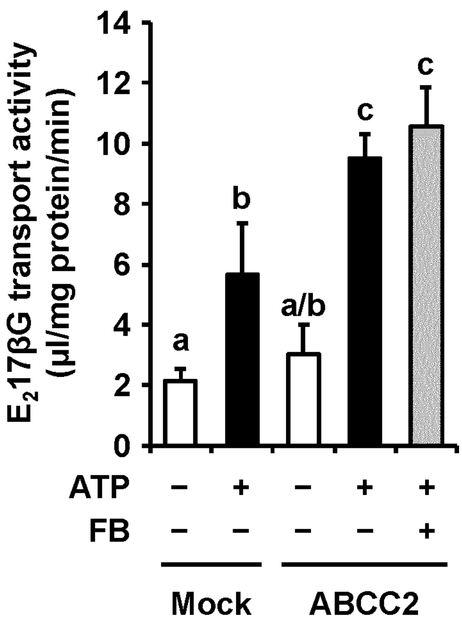
E2 17βG transport activity (µl/mg protein/min)
14
12
10
8
6
4
2
0
a
b
a/b
c
c
ATP – + – + +
FB – – – – +
Mock
ABCC2

Fig. 6

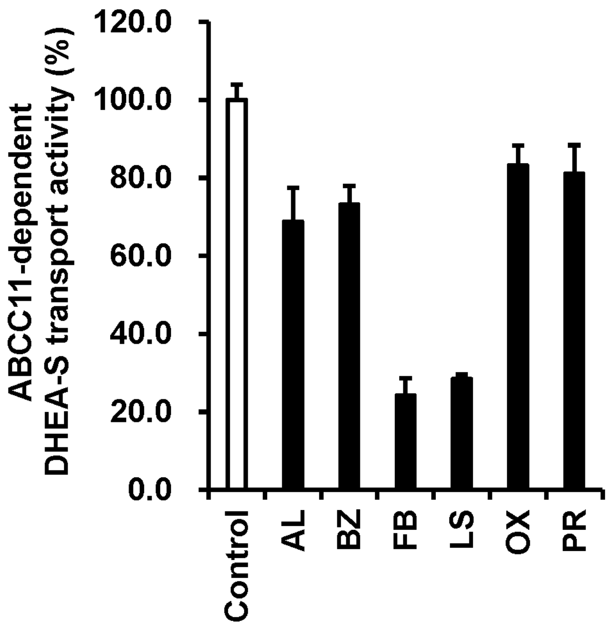
ABCC11-dependent
DHEA-S transport activity (%)
120.0
100.0
80.0
60.0
40.0
20.0
0.0
Control
AL
BZ
FB
LS
OX
PR

Fig. 7

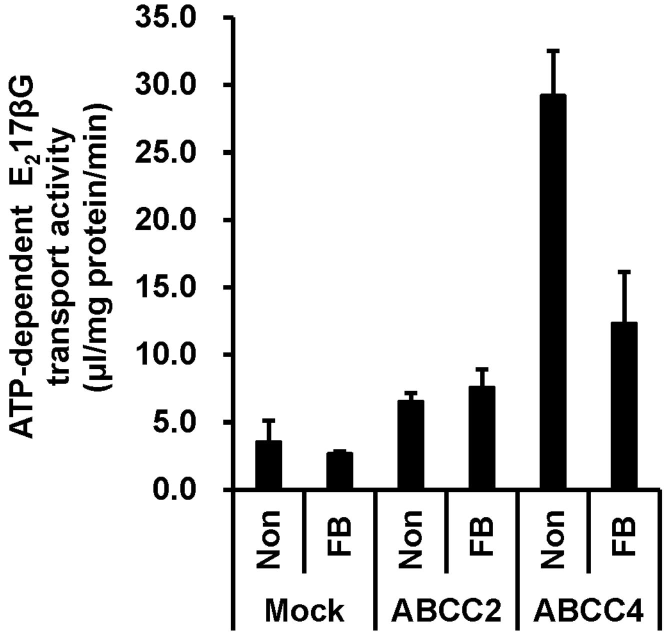
ATP-dependent $E_2 17\beta G$ transport activity (μl/mg protein/min)
35.0
30.0
25.0
20.0
15.0
10.0
5.0
0.0
Non
FB
Non
FB
Non
FB
Mock
ABCC2
ABCC4

Fig. 8

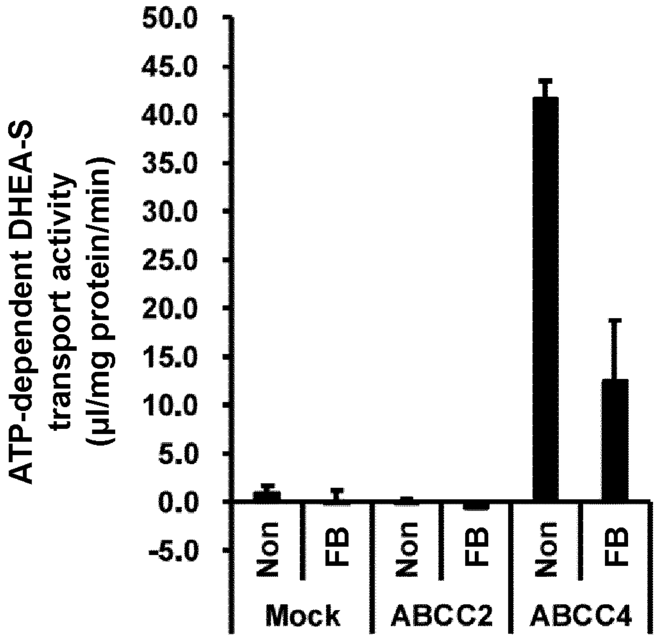
ATP-dependent DHEA-S transport activity (µl/mg protein/min)
50.0
45.0
40.0
35.0
30.0
25.0
20.0
15.0
10.0
5.0
0.0
-5.0
Non
FB
Non
FB
Non
FB
Mock
ABCC2
ABCC4

## INTERNATIONAL SEARCH REPORT

International application No.
PCT/JP2020/031672

A. CLASSIFICATION OF SUBJECT MATTER
A61K 45/00(2006.01)i; A61P 43/00(2006.01)i; A61P 17/00(2006.01)i; A61Q 15/00(2006.01)i; A61K 8/49(2006.01)i
FI: A61K45/00; A61P17/00; A61P43/00 101; A61K8/49; A61Q15/00
According to International Patent Classification (IPC) or to both national classification and IPC

B. FIELDS SEARCHED
Minimum documentation searched (classification system followed by classification symbols)
A61K45/00; A61P43/00; A61P17/00; A61Q15/00; A61K8/49

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII); CAplus/REGISTRY/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X<br>A | EL-SHEIKH, AAK, et al., "Effect of hypouricaemic and hyperuricaemic drugs on the renal urate efflux transporter, multidrug resistance protein 4", British Journal of Pharmacology, 25 August 2008, vol. 155, pp. 1066-1075 in particular, abstract, fig. 2, 6, table 1 | 1, 13-14<br>2-12, 15-17 |
| X<br>A | WO 2007/148835 A1 (NIPPON CHEMIPHAR CO., LTD.) 27.12.2007 (2007-12-27) claim 4, table 1 | 14<br>1-13, 15-17 |
| X<br>A | SHEN, Zancong, et al., "In Vitro and In Vivo Interaction Studies Between Lesinurad, a Selective Urate Reabsorption Inhibitor, and Major Liver or Kidney Transporters", Clin. Drug Investig., 07 March 2016, vol. 36, pp. 443-452 in particular, abstract, table 1 | 14<br>1-13, 15-17 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 September 2020 (07.09.2020) | 24 September 2020 (24.09.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2020/031672

C (Continuation). DOCUMENTS CONSIDERED TO BE RELEVANT

| Category* | Citation of document, with indication, where appropriate, of the relevant passages | Relevant to claim No. |
|---|---|---|
| A | KEPPLER, Dietrich, "Multi drug Resistance Proteins (MRPs, ABCCs): Importance for Pathophysiology and Drug Therapy", Handbook of Experimental Pharmacology, 26 October 2010, vol. 201, pp. 299-323 in particular, abstract, page 311, lines 20-24, page 312, line 5 from the bottom to page 313, line 2 | 1-17 |
| A | WO 2018/023126 A1 (KASPAR, Roger L.) 01.02.2018 (2018-02-01) claim 1, examples | 1-17 |

Form PCT/ISA/210 (continuation of second sheet) (January 2015)

**INTERNATIONAL SEARCH REPORT**
Information on patent family members

International application No.
PCT/JP2020/031672

| Patent Documents referred in the Report | Publication Date | Patent Family | Publication Date |
|---|---|---|---|
| WO 2007/148835 A1 | 27 Dec. 2007 | US 2010/0234399 A1 claim 4, table 1 | |
| WO 2018/023126 A1 | 01 Feb. 2018 | JP 2019-523302 A claim 1, examples | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2725886 B
- US 62890053


### Non-patent literature cited in the description


- **INOUE Y. et al.** *Journal of plastic, reconstructive & aesthetic surgery: JPRAS,* 2010, vol. 63, 1369-1374
- **ISHIKAWA T. et al.** *Frontiers in genetics,* 2013, vol. 3, 306
- **TOYODA Y. et al.** *FASEB journal: Official publication of the Federation of American Societies for Experimental Biology,* 2009, vol. 23, 2001-2013
- **MARTIN A. et al.** *J. Invest. Dermatol.,* 2010, vol. 130, 529-540
- **TOYODA Y. et al.** *Xenobiotica,* July 2008, vol. 38 (7-8), 833-62
- **TOYODA Y. et al.** *Int. J. Mol. Sci.,* 2017, vol. 18, 417
- **UEMURA, T. et al.** *Cancer Sci.,* 2010, vol. 101 (11), 2404-2410
- **TOYODA Y. et al.** *Journal of Dermatology,* 2020
- **KHOSRAVAN R. et al.** *Clin. Pharmacokinet.,* 2006, vol. 45, 821-841
- **TOYODA Y. et al.** *International Journal of Molecular Sciences,* 2017, vol. 18, 417
- **CHEN ZS. et al.** *Mol. Pharmacol.,* 2005, vol. 67, 545-557
- **TOYODA Y. et al.** *Scientific Reports,* 2016, vol. 6, 24586